(19) Europäisches Patentamt
European Patent Office
Office européen des brevets

(11) Veröffentlichungsnummer: **0 365 836**
**A1**

(12) # EUROPÄISCHE PATENTANMELDUNG

(21) Anmeldenummer: **89117605.9**

(22) Anmeldetag: **23.09.89**

(51) Int. Cl.5: **C12N 15/53 , C12N 9/02 , C12Q 1/26**

(30) Priorität: **03.10.88 DE 3833601**

(43) Veröffentlichungstag der Anmeldung: **02.05.90 Patentblatt 90/18**

(84) Benannte Vertragsstaaten: **AT BE CH DE ES FR GB GR IT LI LU NL SE**

(71) Anmelder: **BOEHRINGER MANNHEIM GMBH**
**Sandhofer Strasse 116**
**D-6800 Mannheim 31(DE)**

(72) Erfinder: **Schumacher, Günther, Dr. rer. nat.**
**Kapellenweg 20**
**D-8139 Bernried(DE)**
Erfinder: **Moellering, Hans**
**Herrestrasse 10**
**D-8132 Tutzing(DE)**

(54) **Pyruvatoxidase-Mutanten.**

(57) Pyruvatoxidase, die mindestens einen Austausch der Aminosäure 178 und/oder der Aminosäure 425 in der Aminosäuresequenz des Wildtyp-Enzyms enthält und stabiler als das Wildtyp-Enzym ist. Zu ihrer Herstellung wird eine rekombinante DNA verwendet, welche mindestens einen Austausch von Base 532, 533, 534, 1273, 1274 und/oder 1275 enthält.

**EP 0 365 836 A1**

# Pyruvatoxidase-Mutanten

Die Erfindung betrifft Mutanten der Pyruvatoxidase, welche stabiler sind als das Wildtypenzym und deshalb besser zur enzymatischen Bestimmung von Pyruvat und Pyruvat-liefernden Reaktionen geeignet sind. Pyruvatoxidase (E.C. 1.2.3.3) ist ein Enzym, welches Pyruvat in Gegenwart von Phosphationen und Sauerstoff decarboxyliert, unter Bildung von $H_2O_2$ (Federation Proceedings 13 (1954), 734 - 738). Von den Reaktionsprodukten Acetylphosphat $CO_2$ und $H_2O_2$ ist besonders Letzteres analytisch gut erfaßbar, und daher eignet sich dieses Enzym für die quantitative Bestimmung von Pyruvat und von Pyruvat-bildenden Enzymen bzw. deren Substraten.

Aus EP-A 0 117 550 ist eine Pyruvatoxidase bekannt, welche ohne Zusatz von FAD, Thiamin-Pyrophosphat (TPP) und zweiwertigen Metallionen aktiv ist. Dieses Enzym bildet in Gegenwart von Serum und Magnesiumionen keine unlöslichen Niederschläge und besitzt eine ausgezeichnete Lagerstabilität. Es zeigt sich jedoch, daß bei hohen Salzkonzentrationen des Serums sowie bei pH-Werten von > 7 diese Pyruvatoxidase nur eine begrenzte Stabilität aufweist. Überraschenderweise wurden nun Mutanten der Pyruvatoxidase aus Lactobacillus plantarum (DSM 2571) gefunden, die eine erhöhte Stabilität gegenüber Salz und im alkalischen pH-Bereich aufweisen.

Gegenstand der Erfindung ist daher eine Pyruvatoxidase, welche Pyruvat unter Bildung von $H_2O_2$ decarboxyliert und ohne Zusatz von FAD, TPP und zweiwertigen Metallionen aktiv ist, die dadurch gekennzeichnet ist, daß sie in der Aminosäuresequenz mindestens einen Austausch von Prolin in der Position 178 und/oder von Alanin in der Position 425 enthält. Bevorzugt ist ein Mutantenenzym, bei dem Prolin 178 gegen Serin und/oder Alanin 425 gegen Valin ausgetauscht ist.

Das Mutantenenzym ist charakterisiert durch ein Molekulargewicht von 250000 (bestimmt in der Ultrazentrifuge nach Ames), einem pH-Optimum von 6,5 und einer $K_m$ mit Pyruvat (25°C) von ca. 0,4 mmol/l, einer $K_m$ mit Phosphat (25°C) von ca. 2,3 mmol/l.

Das Mutantenenzym besitzt in 0,1 mol/l Kaliumphosphatpuffer pH 8, mit 0,15 mol/l NaCl nach 30 Minuten bei 25°C eine Restaktivität von mindestens 45 %.

Ein bevorzugtes Mutantenenzym weist unter diesen Bedingungen eine Restaktivität von mindestens 70 % auf. Ein besonders bevorzugtes Mutantenenzym weist eine Restaktivität von mindestens 85 % auf.

Die Herstellung der erfindungsgemäßen Mutantenenzyme erfolgt, indem man nach bekannten gentechnologischen Methoden eine rekombinante DNA, welche ein Pyruvatoxidase-Gen mit im wesentlichen der Sequenz des Wildtypenzyms (Fig. 1), aber zusätzlich mindestens einen Austausch von Base 532, 533, 534, 1273, 1274 und/oder 1275 enthält, in einen Expressionsvektor einbaut, mit diesem einen geeigneten Wirtsstamm transformiert, auf den Expressionsvektor selektioniert, den so transformierten Wirtsstamm unter geeigneten Bedingungen anzieht und aus dem Ansatz das erfindungsgemäße Mutantenenzym gewinnt.

Vorzugsweise wird als rekombinante DNA eine DNA verwendet, welche einen Austausch von C gegen T bei Base 532 und/oder bei Base 1274 enthält.

Ein weiterer Gegenstand der Erfindung ist eine rekombinante DNA, welche ein Pyruvatoxidasegen enthält, welches mindestens einen Austausch von Base 532, 533, 534, 1273, 1274 und/oder 1275 enthält. Bevorzugt ist Base 532 und/oder Base 1274 ausgetauscht. Besonders bevorzugt ist der Austausch dieser Base(n) gegen T.

Es ist aber auch jeder andere Austausch möglich, der dazu führt, daß das Codon 532, 533, 534 für eine andere Aminosäure als Prolin codiert und/oder daß das Codon 1273, 1274, 1275 für eine andere Aminosäure als Alanin codiert.

Besonders bevorzugt wird als rekombinante DNA eines der Plasmide pBP 201, pBP 202, pBP 203, pBP 203a oder pBP 2006 verwendet. Als Wirtssysteme sind grampositive und gramnegative Mikroorganismen, wie Bacillus spec. oder E.coli, geeignet. Bevorzugt werden Mikroorganismen der Gattung E.coli verwendet. Besonders bevorzugt werden die Mikroorganismen E.coli Laql$^q$, DSM 3689, ED 8654, DSM 2102, E.coli, DSM 4105 und E.coli, DSM 4106, verwendet. Als Expressionsplasmide sind insbesondere Vektoren wie pBR 322 und Derivate geeignet.

Weitere Gegenstände der Erfindung sind die Plasmide pBP 201, pBP 202, pBP 203, pBP 203a und pBP 2006. Diese enthalten das Pyruvatoxidase-Gen des Wildtyps (Fig. 1), wobei auf DNA-Ebene folgende Austausche vorliegen:

| | Base 532 von C in T | Base 1274 von C in T | Austausch im 500 bp BanII/EcoRV Fragment |
|---|---|---|---|
| pBP 201 | + | - | - |
| pBP 202 | + | - | + |
| pBP 203 | + | + | - |
| pBP 203a | - | + | - |
| pBP 2006 | + | + | + |

Ein weiterer Gegenstand der Erfindung ist die DNA-Sequenz der Wildtyp-Pyruvatoxidase. Diese DNA ist in plasmid pBP200 enthalten und geeignet als Ausgangsprodukt für die Herstellung der erfindungsgemäßen rekombinanten DNA, welche das Gen der Pyruvatoxidase-Mutante enthält.

Ein weiterer Gegenstand der Erfindung ist die Verwendung der erfindungsgemäßen Mutantenenzyme zur Bestimmung von Pyruvat und von Pyruvat-bildenden Enzymen bzw. deren Substraten.

Dabei erfolgt die Bestimmung von Pyruvat insbesondere durch Messung von gebildetem $H_2O_2$. Hierfür sind zahlreiche geeignete Methoden bekannt, die hier nicht gesondert beschrieben werden müssen. Ebenfalls gut geeignet ist die Messung des Sauerstoffverbrauchs, beispielsweise mittels Sauerstoffelektrode.

Typische Beispiele für Bestimmungen, die mit dem erfindungsgemäßen Enzym durchgeführt werden können, sind in EP-A 0274 425 beschrieben und sind z. B.:

Glutamat-Pyruvat-Transaminase oder Alpha-Ketoglutarat
Glutamat-Oxalacetat-Transaminase
Pyruvatkinase oder ADP
Lactatdehydrogenase oder Milchsäure
Glycerin oder Glycerinphosphatkinase
Triglycerid
Creatin-Phosphokinase oder Creatin
Myokinase, Thiokinase oder Fettsäure.

Ein weiterer Gegenstand der Erfindung ist ein Reagenz zur Bestimmung von Pyruvat, welches dadurch gekennzeichnet ist, daß es erfindungsgemäße Mutantenenzyme, Phosphat, ein System zur Bestimmung von $H_2O_2$ und ggf. ein System zur Bildung von Pyruvat enthält.

Als Puffer läßt sich jede geeignete Puffersubstanz verwenden, die im pH-Bereich zwischen ca. 5 und 9 Puffern liegt. Gut geeignet ist insbesondere Phosphatpuffer, für den ein gesonderter Pufferzusatz neben dem für die Reaktion erforderlichen Phosphat nicht mehr nötig ist. Bei der Auswahl des Puffers ist zusätzlich zu berücksichtigen, welche pH-Werte für die im System ebenfalls vorhandenen Hilfsenzyme und beispielsweise Chromophore erforderlich sind. An Hand der bekannten Daten dieser Enzyme kann der Fachmann jedoch ohne weiteres die geeignete Auswahl des Puffers treffen.

Das erfindungsgemäße Reagenz eignet sich ebenso zur Imprägnierung von Trägermaterialien, wie Papier, Kunststoff, Folien und dergleichen für Teststreifen.

Vorzugsweise enthält das erfindungsgemäße Reagenz
1 - 50 U/ml Mutantenenzym
10 - 500 mmol/l Phosphat, pH 6 - 8

Die folgenden Beispiele erläutern die Erfindung weiter.

Beispiel 1

Zellen von E.coli ED lacI$^q$, DSM 3689, welche die Plasmide pBP 201, pBP 202, pBP 203, pBP 203a oder pBP 2006 enthalten, werden über Nacht bei 28°C bis 30°C im 1 Liter-Fermenter angezogen. Als Medium wird ein Vollmedium (Hefe/Peptonextrakt) verwendet, welches 0,4 % Lactose und 100 mmol/l Phosphat 7,5 enthält. Nach 15minütiger Zentrifugation bei 8000 rpm werden die Zellen mit Lysozym aufgeschlossen und die Pyruvatoxidase über DEAE-Sephadex und Gelfiltration (Sephacryl S 200) gereinigt.

Beispiel 2

Überprüfung der Stabilität in Salzlösung

a) Aktivitätsbestimmung

Zur Überprüfung der Stabilität des Enzyms, wird die Aktivität des Enzyms auf Grund folgender Reaktionen bestimmt:

(1) Pyruvat + $P_i$ + $O_2$ $\xrightarrow{Py\text{-}OD}$ Acetyl-P + $CO_2$ + $H_2O_2$

(2) $H_2O_2$ + 4-Aminoantipyrin(4AAP) + 2-Hydroxy-3,5-dichlorbenzolsulfonsäure (HDBS) $\xrightarrow{POD}$ Chinon-Farbstoff (546 nm) + $2H_2O$ + HCl

$H_2O_2$ wird in äquimolarer Menge des Farbstoffes verbraucht.
1 U Py-OD = 1 umol Pyruvat-Umsatz/min bei 25°C.
Die Bestimmung der Aktivität der Pyruvatoxidase (PyOD) erfolgt mit einem Reagenz, bestehend aus (Endkonzentrationen im Test):
72 mmol/l Kaliumphosphatpuffer, pH 6,7; 8 mmol/l 4-AAP; 6,8 mmol/l HDBS, 50 mmol/l Pyruvat, 10 U/ml Peroxidase (POD).
Zu 2 ml dieses Reagenses werden 0,1 ml der zu prüfenden Pyruvatoxidaselösung zugegeben und bei 546 nm die Extinktionsänderung pro Minute ( E/min) bei 25°C und einer Schichtdicke von 1 cm bestimmt ( = 16,5 $cm^2$/umol). Die Aktivität wird berechnet nach:

$$\text{Aktivität (U)} = \frac{E/min \cdot V}{\epsilon}$$

V: Küvettenvolumen ($cm^3$)

b) Belastung bei pH 7,5

Pyruvatoxidase wird in einer Inkubations-Lösung bestehend aus: 0,1 mol/l Kaliumphosphatpuffer pH 7,5 und 0,15 mol/l Natriumchlorid bei 25°C bzw. 37°C über die in der Tabelle I angegebene Zeit belastet und die Aktivität, wie in Beispiel 2a beschrieben, bestimmt. Die Ergebnisse sind aus Tab. I/II zu ersehen. Fig. 2 zeigt die Ergebnisse nach Belastung bei 37°C nach 20 Stunden bei unter schiedlichen pH-Werten in 0,1 mol/l Kalium phosphatpuffer.

Danach zeigt sich, daß die von den erfindungsgemäßen Plasmiden kodierten Pyruvatoxidasen dem Wildtypenzym überlegen sind. Insbesondere die Mutante pBP 2006 weist eine hohe Stabilität gegenüber alkalischem pH- Wert und Salz auf.

Tabelle I

| Enzym | Temperatur 25°C % Restaktivität nach | |
|---|---|---|
| | 10 min | 30 min |
| Wildtyp | 28 | 5 |
| Mutanten | | |
| pBP 201 | 65 | 45 |
| pBP 202 | 89 | 70 |
| pBP 203 | 84 | 76 |
| pBP 2006 | 98 | 86 |

Tabelle II

| Enzym | Temperatur 37°C % Restaktivität nach | | |
|---|---|---|---|
| | 5 min | 10 min | 20 min |
| Wildtyp | 4 | 0 | 0 |
| Mutanten | | | |
| pBP 202 | 48 | 19 | 4 |
| pBP 203 | 45 | 18 | 5 |
| pBP 2006 | 65 | 36 | 34 |

## Beispiel 3

Überprüfung der Stabilität mit unverdünntem Serum

9,5 ml unverdünntes Serum wird durch tropfenweise Zugabe von 10 %iger Essigsäure oder 2 mol/l NaOH auf die in Tabelle III angegebenen pH-Werte eingestellt. Anschließend werden 0,5 ml Pyruvatoxidase-Lösung (150 U/ml) zugegeben und eine Belastung bei 37°C durchgeführt. Die Bestimmung der Restaktivität erfolgt wie in Beispiel 2a beschrieben. Die Ergebnisse zeigt Tabelle III.

Tabelle III

| pH-Wert | % Restaktivität nach | | | | | |
|---|---|---|---|---|---|---|
| | 1 min | 2 min | 5 min | 1 min | 2 min | 5 min |
| | Wildtyp-Enzym | | | pBP 2006 | | |
| 5,92 | 103 | 93,5 | 71,2 | 93,4 | 86,4 | 73,2 |
| 6,45 | 96,0 | 95,1 | 90,2 | 98,1 | 88,6 | 90,6 |
| 6,98 | 90,9 | 71,5 | 17,5 | 92,4 | 82,9 | 77,9 |
| 7,58 | 67,5 | 26,9 | 1,5 | 81,2 | 78,7 | 53,1 |
| 8,08 | 36,2 | 6,5 | - | 87,0 | 78,2 | 21,8 |
| 8,71 | 1,3 | - | - | 90,3 | 78,6 | 31,6 |

Beispiel 4

Überprüfung der Stabilität für Pyruvatoxidase aus Plasmid pBP203a

Kolonien von E.coli Iaql$^q$, DSM 3689, mit und ohne Plasmid pBP203a, werden auf Vollmedium-Plasmid mit Cellulosefiltern über Nacht kultiviert. Anschließend wird mit Chloroform/Toluol lysiert und der Filter 20 Minuten bei 37°C in 0,1 mol/l Kaliumphosphatpuffer pH 7,5 und 0,15 mol/l Natriumchlorid inkubiert. Dann wird der Filter auf Platten gebracht, welche ein Indikatormedium, bestehend aus: 40 mmol/l Natriumpyruvat, 0,24 mg/l 4-Amino-Antipyrin, 1,5 mg/ml N-Ethyl-N-(3-methylphenyl)-2-aminoethansulfonsäure EST, 12,5 ug/ml Peroxidase, 1 % Agar, 50 mmol/l Kaliumphosphatpuffer pH 7,2 enthält und nach einer Minute die Farbreaktion beobachtet. Es zeigt sich, daß nur Kolonien von Mikroorganismen, welche pBP203a enthalten, eine Farbreaktion geben und demzufolge noch Pyruvatoxidase vorhanden ist. Die Mikroorganismen, die das Wildtyp-Plasmid enthalten, zeigen keine Farbreaktion mehr. Dies bedeutet, daß auch die Pyruvat-Oxidase-Mutante aus pBP203a gegenüber dem Wildtyp-Enzym eine überlegene Stabilität aufweist.

Beispiel 5

Herstellung der Pyruvatoxidase-Mutantenenzyme

Ausgehend vom Plasmid pBP 200, (Herstellung nach Beispiel 6), welches das Wildtyp-Gen von Pyruvatoxidase enthält, wird mit der Methode der gerichteten Mutagenese die entsprechende Mutation auf DNA-Ebene durchgeführt. Dieses Verfahren ist im Detail beschrieben in Proc. Nat. Acad. Sci USA 82 (1985), 488 - 492 und Nat. Enzymol. 1987 sowie in Bulletin 1313 von Biorad Laboratories, Richmond USA zu Muta-Gene$^R$ in Vitro-Mutagenesis-Kit.

Zur Herstellung von pBP201 wurde das Oligonucleotid A der Sequenz
5′-CGTTCAGCTGAAATCTGTTG-3′ verwendet.

Zur Herstellung von pBP203a wurde das Oligonucleotid B der Sequenz
5′-AACTTGCGTCACCAAATCTT-3′ verwendet.

Zur Herstellung von pBP203 wurden die Oligonucleotide A und B verwendet.

Plasmid pBP202, welches wie pBP201 einen Austausch von C gegen T bei Base 532 sowie zusätzlich eine Mutation auf einem 500 bp großen BanII EcoRV-Fragment des Wildtypgens enthält, wurde bei der Deutschen Sammlung für Mikroorganismen (DSM) hinterlegt.

Das Plasmid pBP2006 wurde aus pBP202 durch gerichtete Mutagenese, unter Verwendung des Oligonucleotids B hergestellt.

Weitere Mutantenenzyme können hergestellt werden, unter Verwendung von Oligonucleotid C:
5′-CGTTCAGCGCTAATCTGTTG-3′
(Austausch Prolin 178 gegen Serin)
von Oligonucleotid D:

5'CGTTCAGCGACAATCTGTTG-3'
(Austausch Prolin 178 gegen Valin)
von Oligonucleotid E:
5'CGTTCAGCAGCAATCTGTTG-3'
(Austausch Prolin 178 gegen Alanin
von Oligonucleotid F:
5'-AACTTGCGTGGTCAAATCTT-3'
(Austausch Alanin 425 gegen Threonin)
von Oligonucleotid G
5'-AACTTGCGTAAGCAAATCTT-3'
(Austausch Alanin 425 gegen Lysin)
von Oligonucleotid H
5'-AACTTGCGTGCCCAAATCTT-3'
(Austausch Alanin 425 gegen Glycin)

Beispiel 6

Herstellung von pBP200 (DSM 4875)

Plasmid pKK177-3 (DSM 3062) wird mit Eco.RI und Smal gespalten. Aus dem Pyruvatoxidase-codierenden DNA-Fragment (Fig. 1) wird ein ca. 1kb Eco.RI-SalI-Fragment und ein ca. 1,2kb SalI-Eco.RV-Fragment isoliert. Alle drei Fragmente werden miteinander ligiert. Es wird in E.coli (DSM 3689) transformiert und auf Ampicillin-Resistenz selektioniert. Das die Pyruvatoxidase-codierende Plasmid pBP200 trägt die Restriktionsstellen wie in Fig. 3 angegeben.

Plasmid pBP200 ist ebenso erhältlich aus Plasmid pBP202 durch Deletion des ca. 2,2kb großen Eco.RI-Eco.RV-Fragments und Ersatz dieses Fragments durch ein ca. 1kb großes Eco.RI-SalI und ein ca. 1,2kb großes SalI-Eco.RV-Fragment der DNA nach Fig. 1.

**Ansprüche**

1. Pyruvatoxidase, welche Pyruvat unter Bildung von $H_2O_2$ decarboxyliert und ohne Zusatz von FAD, Thiaminpyrophosphat und zweiwertigen Metallionen aktiv ist, dadurch gekennzeichnet, daß sie in der Aminosäuresequenz mindestens einen Austausch von Prolin in der Position 178 und/oder Alanin in der Position 425 enthält.

2. Pyruvatoxidase nach Anspruch 1, dadurch gekennzeichnet, daß in Position 178 der Aminosäuresequenz Prolin gegen Serin und/oder in Position 425 Alanin gegen Valin ausgetauscht ist.

3. Pyruvatoxidase nach den Ansprüchen 1 oder 2, dadurch gekennzeichnet, daß sie ein Molekulargewicht von 250000, ein pH-Optimum von 6,5, eine $K_m$ mit Pyruvat (25°C) von ca. 0,4 mmol/l und einer $K_m$ mit Phosphat (25°C) von ca. 2,3 mmol/l aufweist.

4. Rekombinante DNA, welche ein Pyruvatoxidase-Gen enthält, welches mindestens einen Austausch von Base 532, 533, 534, 1273, 1274 und/oder 1275 enthält.

5. Verfahren zur Herstellung von Pyruvatoxidase, gem. den Ansprüchen 1 - 3, dadurch gekennzeichnet, daß man eine rekombinante DNA, welche ein Pyruvatoxidase-Gen enthält, welches mindestens einen Austausch von Base 532, 533, 534, 1273, 1274 und/oder 1275 enthält, in einen geeigneten Wirtsstamm transformiert, auf den Expressionsvektor selektioniert, den so transformierten Wirtsstamm unter geeigneten Bedingungen anzieht und aus dem Ansatz die Pyruvatoxidase gewinnt.

6. Verwendung eines Enzyms nach den Ansprüchen 1 - 3 zur Bestimmung von Pyruvat durch Messung von gebildetem $H_2O_2$, $CO_2$ oder Acetylphosphat oder durch Messung des Sauerstoffverbrauchs.

7. Reagenz zur Bestimmung von Pyruvat, dadurch gekennzeichnet, daß es Pyruvatoxidase gemäß den Ansprüchen 1 - 3 und ein System zur Bestimmung von $H_2O_2$ enthält.

8. DNA-Sequenz, welche für die Aminosäure-Sequenz gemäß Fig. 1 codiert.

9. DNA-Sequenz gemäß Fig. 1

10. Verwendung einer DNA-Sequenz nach Anspruch 15 oder 16 zur Herstellung von Pyruvat-Oxidasemutanten.

Fig. 1

```
                 10                                30                                50
  1   ATGGTTATGAAACAAACAAAACAAACTAACATACTAGCAGGTGCAGCAGTTATTAAAGTT
      MetValMetLysGlnThrLysGlnThrAsnIleLeuAlaGlyAlaAlaValIleLysVal

                 70                                90                               110
 61   TTAGAAGCTTGGGGAGTAGATCATTTGTATGGTATTCCTGGAGGTTCAATTAATTCAATT
      LeuGluAlaTrpGlyValAspHisLeuTyrGlyIleProGlyGlySerIleAsnSerIle

                130                               150                               170
121   ATGGACGCATTATCAGCAGAAAGGGATCGAATCCATTATATTCAAGTACGGCATGAAGAA
      MetAspAlaLeuSerAlaGluArgAspArgIleHisTyrIleGlnValArgHisGluGlu

                190                               210                               230
181   GTTGGTGCAATGGCCGCCGCTGCTGATGCTAAGCTAACGGGTAAAATCGGGGTTTGCTTC
      ValGlyAlaMetAlaAlaAlaAlaAspAlaLysLeuThrGlyLysIleGlyValCysPhe

                250                               270                               290
241   GGCTCAGCGGGACCTGGTGGCACTCATCTTATGAATGGGTTATATGATGCGCGTGAAGAC
      GlySerAlaGlyProGlyGlyThrHisLeuMetAsnGlyLeuTyrAspAlaArgGluAsp

                310                               330                               350
301   CATGTCCCTGTTCTAGCACTTATTGGTCAATTTGGAACTACTGGGATGAACATGGATACG
      HisValProValLeuAlaLeuIleGlyGlnPheGlyThrThrGlyMetAsnMetAspThr

                370                               390                               410
361   TTCCAAGAAATGAATGAGAATCCGATTTATGCGGACGTTGCAGATTATAATGTAACAGCC
      PheGlnGluMetAsnGluAsnProIleTyrAlaAspValAlaAspTyrAsnValThrAla

                430                               450                               470
421   GTCAATGCTGCCACGTTGCCACATGTTATTGACGAAGCAATTCGACGCGCCTACGCGCAC
      ValAsnAlaAlaThrLeuProHisValIleAspGluAlaIleArgArgAlaTyrAlaHis

                490                               510                               530
481   CAAGGTGTTGCGGTTGTGCAAATTCCAGTCGATTTACCATGGCAACAGATTCCAGCTGAA
      GlnGlyValAlaValValGlnIleProValAspLeuProTrpGlnGlnIleProAlaGlu

                550                               570                               590
541   CGGCCACTTATTTACTATGGCATTGGAGCTCGTAAGGCTGGTAAAGAACTCGAACAATTG
      ArgProLeuIleTyrTyrGlyIleGlyAlaArgLysAlaGlyLysGluLeuGluGlnLeu

                610                               630                               650
601   AGTAAAACGTTGAAAATTCCATTAATGAGTACGTATCCAGCTAAGGGTATTGTCGCGGAT
      SerLysThrLeuLysIleProLeuMetSerThrTyrProAlaLysGlyIleValAlaAsp

                670                               690                               710
661   CGTTATCCAGCCTATTTGGGTTCTGCTAATCGGGTGGCACAAAAACCGGCGAATGAGGCA
      ArgTyrProAlaTyrLeuGlySerAlaAsnArgValAlaGlnLysProAlaAsnGluAla

                730                               750                               770
721   CTTGCGCAAGCCGACGTTGTTTTATTTGTTGGTAATAATTATCCGTTTGCAGAAGTTTCC
      LeuAlaGlnAlaAspValValLeuPheValGlyAsnAsnTyrProPheAlaGluValSer

                790                               810                               830
781   AAAGCGTTTAAAAATACGCGTTATTTCTTACAAATTGATATTGATCCGGCTAAGTTAGGT
      LysAlaPheLysAsnThrArgTyrPheLeuGlnIleAspIleAspProAlaLysLeuGly
```

Fig. 1 (Fortsetzung)

```
        850                 870                 890
841     AAACGACATAAAACAGATATTGCGGTACTTGCTGATGCACAAAAGACGCTGGCTGCAATT
        LysArgHisLysThrAspIleAlaValLeuAlaAspAlaGlnLysThrLeuAlaAlaIle

        910                 930                 950
901     TTAGCACAGGTATCTGAACGGGAGTCGACACCTTGGTGGCAAGCCAATTTAGCCAATGTT
        LeuAlaGlnValSerGluArgGluSerThrProTrpTrpGlnAlaAsnLeuAlaAsnVal

        970                 990                 1010
961     AAAAATTGGCGGGCTTATCTAGCTTCATTAGAAGATAAGCAGGAAGGGCCTTTACAAGCA
        LysAsnTrpArgAlaTyrLeuAlaSerLeuGluAspLysGlnGluGlyProLeuGlnAla

        1030                1050                1070
1021    TATCAAGTGCTACGTGCGGTTAATAAAATTGCGGAGCCTGATGCAATCTATTCGATTGAT
        TyrGlnValLeuArgAlaValAsnLysIleAlaGluProAspAlaIleTyrSerIleAsp

        1090                1110                1130
1081    GTTGGTGATATCAATTTGAATGCGAATCGACATTTGAAATTAACGCCATCCAATCGGCAC
        ValGlyAspIleAsnLeuAsnAlaAsnArgHisLeuLysLeuThrProSerAsnArgHis

        1150                1170                1190
1141    ATTACTTCTAACTTATTTGCTACGATGGGAGTTGGTATTCCGGGAGCAATTGCTGCCAAA
        IleThrSerAsnLeuPheAlaThrMetGlyValGlyIleProGlyAlaIleAlaAlaLys

        1210                1230                1250
1201    CTTAATTATCCTGAGCGGCAGGTGTTTAATCTGGCCGGTGATGGTGGCGCTTCGATGACC
        LeuAsnTyrProGluArgGlnValPheAsnLeuAlaGlyAspGlyGlyAlaSerMetThr

        1270                1290                1310
1261    ATGCAAGATTTGGCGACGCAAGTTCAATACCATTTACCAGTGATTAATGTTGTTTTCACC
        MetGlnAspLeuAlaThrGlnValGlnTyrHisLeuProValIleAsnValValPheThr

        1330                1350                1370
1321    AACTGCCAATATGGATTTATCAAAGATGAGCAGGAAGATACTAATCAGAATGATTTTATT
        AsnCysGlnTyrGlyPheIleLysAspGluGlnGluAspThrAsnGlnAsnAspPheIle

        1390                1410                1430
1381    GGCGTTGAATTCAATGATATTGATTTTAGTAAGATTGCCGATGGCGTGCACATGCAAGCT
        GlyValGluPheAsnAspIleAspPheSerLysIleAlaAspGlyValHisMetGlnAla

        1450                1470                1490
1441    TTTCGAGTTAATAAGATTGAGCAATTACCTGATGTTTTTGAACAAGCCAAAGCAATCGCT
        PheArgValAsnLysIleGluGlnLeuProAspValPheGluGlnAlaLysAlaIleAla

        1510                1530                1550
1501    CAGCATGAACCAGTTCTGATTGATGCGGTGATTACAGGAGATCGGCCACTGCCTGCTGAA
        GlnHisGluProValLeuIleAspAlaValIleThrGlyAspArgProLeuProAlaGlu

        1570                1590                1610
1561    AAGCTTCGTTTAGATTCGGCAATGAGTTCGGCAGCTGATATTGAAGCATTTAAACAACGG
        LysLeuArgLeuAspSerAlaMetSerSerAlaAlaAspIleGluAlaPheLysGlnArg

        1630                1650                1670
1621    TATGAAGCTCAAGATTTACAACCACTTTCAACTTATTTAAAACAATTTGGCTTAGATGAT
        TyrGluAlaGlnAspLeuGlnProLeuSerThrTyrLeuLysGlnPheGlyLeuAspAsp

        1690                1710
1681    TTGCAACATCAAATTGGACAGGGTGGGTTTTAA   1713
        LeuGlnHisGlnIleGlyGlnGlyGlyPheEnd
```

Fig. 2

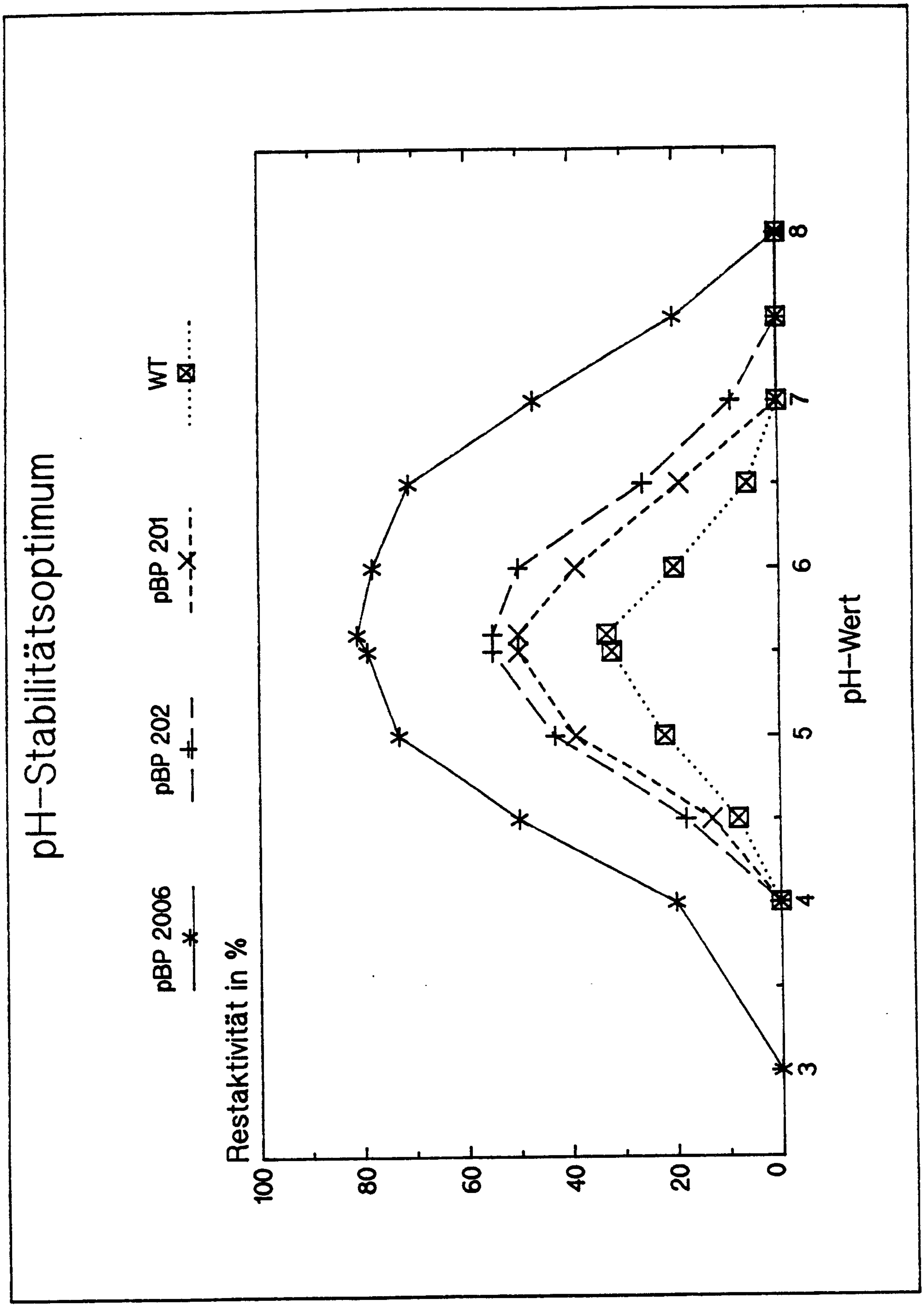
pH-Stabilitätsoptimum
pBP 2006
pBP 202
pBP 201
WT
Restaktivität in %
100
80
60
40
20
0
3
4
5
6
7
8
pH-Wert

Fig. 3

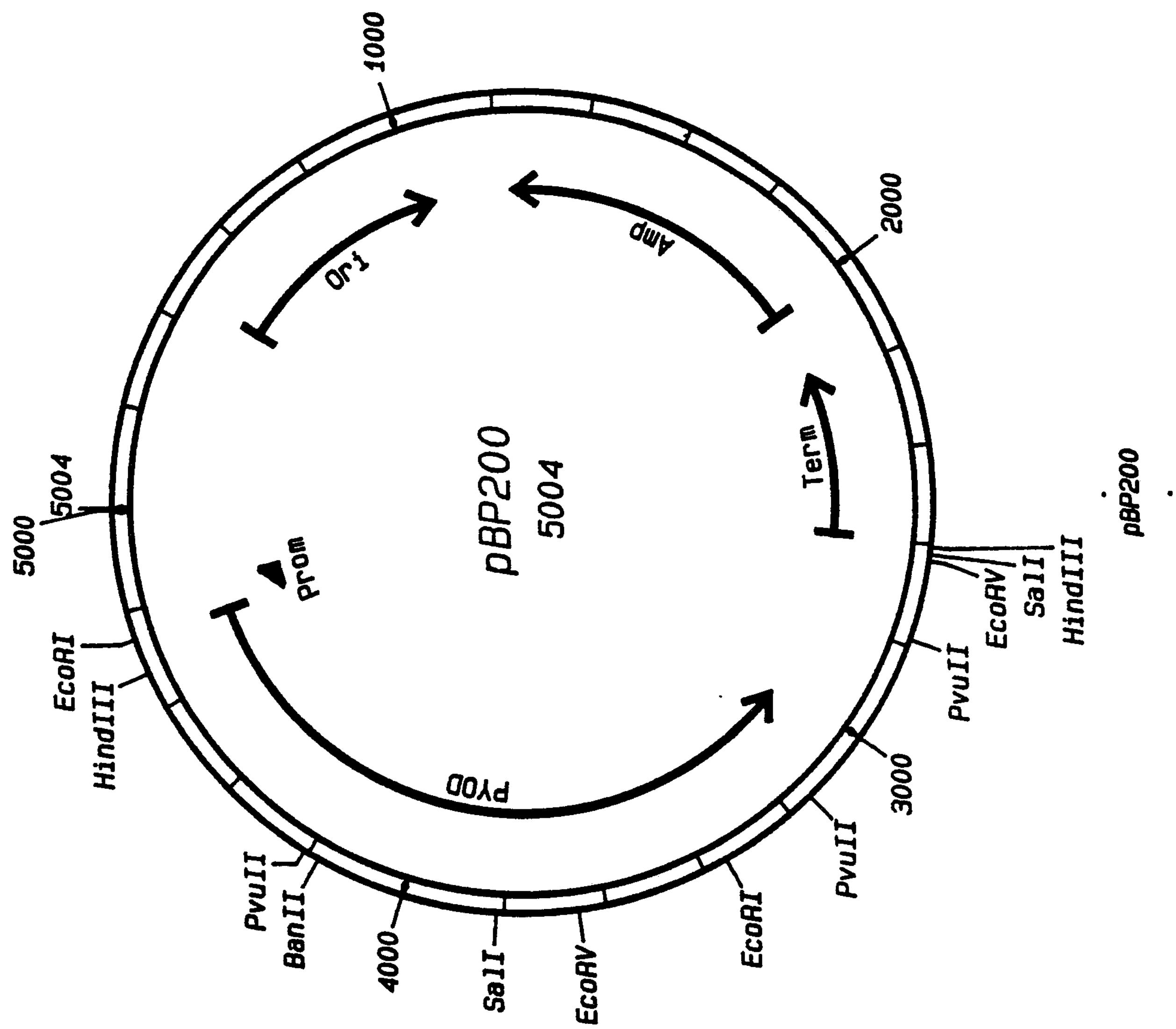
pBP200
5004
Ori
Amp
Term
Prom
pYOD
1000
2000
3000
4000
5000
5004
EcoRV
SalI
HindIII
PvuII
PvuII
EcoRI
EcoRV
SalI
BanII
PvuII
HindIII
EcoRI
pBP200

Europäisches Patentamt

# EUROPÄISCHER RECHERCHENBERICHT

Nummer der Anmeldung

EP 89 11 7605

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.5) |
|---|---|---|---|
| Y,D | EP-A-0 117 550 (BOEHRINGER MANNHEIM GmbH)<br>* Ganzes Dokument * | 8-10 | C 12 N 15/53<br>C 12 N 9/02<br>C 12 Q 1/26 |
| Y,D | EP-A-0 274 425 (TOYO JOZO K.K.)<br>* Beispiel 1-3 * | 8-10 | |
| A | BIOCHEMISTRY, Band 25, 1986, Seiten 3748-3751, American Chemical Society, Washington, DC, US; C. GRABAU et al.: "In Vivo Function of Escherichia coli Pyruvate Oxidase Specifically Requires a Functional Lipid Binding Site" | | |
| A | PROTEIN ENGINEERING, Band 2, Nr. 2, Juli 1988, Seiten 147-152, Eynsham, Oxford, GB; M.J. McPHERSON et al.: "Multiple interactions of lysine-128 of Escherichia coli glutamate dehydrogenase revealed by site-directed mutagenesis studies" | | |
| | | | RECHERCHIERTE SACHGEBIETE (Int. Cl.5) |
| | | | C 12 N |

Der vorliegende Recherchenbericht wurde für alle Patentansprüche erstellt

| Recherchenort | Abschlußdatum der Recherche | Prüfer |
|---|---|---|
| DEN HAAG | 19-01-1990 | VAN PUTTEN A.J. |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPO FORM 1503 03.82 (P0403)